# EUROPEAN PATENT APPLICATION

(11) **EP 2 915 798 A1**
(43) Date of publication of application: **09.09.2015**
(21) Application number: 14157835.1
(22) Date of filing: 05.03.2014
(51) Int. Cl.: C07C 51/41, C07C 51/43, C07C 53/122, C07C 55/10

(54) **Process for manufacturing solid propionate salt**

(71) Applicant: PURAC Biochem BV, 4206 AC Gorinchem (NL)
(72) Inventor: de Haan, André Banier, 4206 AC GORINCHEM (NL); Radenovic, Neda, 4206 AC GORINCHEM (NL)
(74) Representative: Hesselink, Dinah Elisabeth

(57) **Abstract**

The invention pertains to a process for manufacturing a solid propionate salt from a mixture comprising propionate and succinate comprising the steps of
- providing an aqueous medium comprising succinate anions and propionate anions,
- providing an inorganic soluble salt in the aqueous medium in such an amount that the majority of succinate ions is converted to solid succinate salt while the majority of propionate ions remains in solution,
- separating the aqueous medium comprising the majority of propionate ions from the solid succinate salt,
- increasing the concentration of salt in the aqueous medium to convert the majority of propionate ions to solid propionate salt,
- separating the solid propionate salt from the aqueous medium.

It has been found that with the process according to the invention a solid propionate salt can be obtained which has a high purity. More specifically, it has a low succinate content and a low content of organic contaminants.

## Description

The present invention pertains to a process for manufacturing a solid propionate salt from a mixture comprising propionate and succinate, and optionally further contaminants. The present invention also pertains to an integrated fermentation purification process, which comprises a step of obtaining a solid propionate salt.

Propionic acid can be manufactured through fermentation of a carbon source using a microorganism. For example, Zhu et al (Bioresource Technology 101 (2010) 8902-8902) describes a fermentation process for the manufacture of calcium propionate wherein glycerol is fermented using *Propionibacterium acidipropionici*. Calcium hydroxide is added during the fermentation to neutralize the acid generated during the fermentation, resulting in the formation of calcium propionate.

As is known in the art, once the fermentation is completed, the fermentation broth is subjected to purification steps, to yield the acid in the desired form, e.g., in the form of an acid or in the form of the corresponding salt.

Solid propionate salts are attractive for many purposes. For example they are attractive for use in feed and food preservation, where they provide long lasting broad spectrum activity. Most important salts in this respect are calcium, sodium, potassium, and ammonium salts. Calcium salt is considered of particular interest.

A problem which has been found to occur in the preparation of solid propionate salts is that it is difficult to separate the propionate from the succinate which is often formed as side product in propionate fermentations. There is therefore need in the art for a process which makes it possible to manufacture a high-purity solid propionate salt from a mixture comprising propionate and succinate. The present invention provides such a process.

The present invention thus pertains to a process for manufacturing a solid propionate salt from a mixture comprising propionate and succinate comprising the steps of
- providing an aqueous medium comprising succinate anions and propionate anions,
- providing an inorganic soluble salt in the aqueous medium in such an amount that the majority of succinate ions is converted to solid succinate salt while the majority of propionate ions remains in solution,
- separating the aqueous medium comprising the majority of propionate ions from the solid succinate salt,
- increasing the concentration of salt in the aqueous medium to convert the majority of propionate ions to solid propionate salt,
- separating the solid propionate salt from the aqueous medium.

It has been found that with the process according to the invention a solid propionate salt can be obtained which has a high purity. More specifically, it has a low succinate content and a low content of organic contaminants.

Not wishing to be bound by theory, it is believed that the effect of the invention resides in the following. When, to separate the two compounds, one attempts to perform sequential precipitation of calcium succinate and calcium propionate in the absence of inorganic soluble salt, the calcium propionate inhibits and significantly delays the nucleation and crystallization of calcium succinate. This results in a difficult separation of calcium succinate from calcium propionate. More specifically, to cause the calcium succinate to precipitate, it is necessary to go to very high concentrations, where calcium propionate also starts to precipitate, sometimes even before calcium succinate.
In the process of the invention where an inorganic soluble salt is present, succinate precipitation starts faster and further removed from the solubility limit of calcium propionate, leading to preferential precipitation of calcium succinate in a first step. Therefore, the process according to the invention results in an efficient separation. Further, it has been found that the presence of salt also decreases the solubility and promotes nucleation of calcium propionate, resulting in turn in an efficient precipitation of this compound in a second step.

The invention will be described in more detail below.

The first step in the process according to the invention is the provision of an aqueous medium comprising succinate anions and propionate anions.

The concentration and ratio between the various compounds will depend on the source of the aqueous medium.
In one embodiment the weight ratio between propionate and succinate, calculated as the respective salt to be precipitated, is in the range of 25:1 to 1:1, in particular 15:1 to 1:1, more in particular 10:1 to 3:1, still more in particular 8:1 to 3:1. This is the type of ratio that is often obtained in a propionate fermentation process. In one embodiment, the aqueous medium also comprises acetate, a compound which also often formed in propionate fermentations. The weight ratio of acetate to succinate, calculated as the respective salt to be precipitated, may be in the range of 0:1 to 3:1, in particular 0:1 to 1:1, more in particular in the range of 0:1 to 0.5:1, still more in particular in the range of 0:1 to 0.3:1. In one embodiment, the weight ratio of acetate to succinate is in the range of 0.01:1 to 0.3:1.

It is preferred for the aqueous medium to have a propionate concentration in the range of 10-30 wt.%. If the propionate concentration is too low, large amounts of salt will have to be added to the solution, and large volumes of water have to be processed. On the other hand, a very high propionate concentrations is less preferred, because this entails the risk of precipitation of propionate salt. The propionate concentration preferably is in the range of 15 to 25 wt.%, more preferably in the range of 18-23 wt.%.
The succinate concentration in the medium is of lesser importance. It is related to the propionate concentration and the propionate/succinate ratio as specified above. For example, the succinate concentration may be in the range of 0.5-10 wt.%, in particular 1-6 wt.%.
The acetate concentration is also of lesser importance. It may, e.g., be in the range of 0-2 wt.%.
The above concentrations apply to the aqueous medium which will be subjected to the precipitation step. This medium can be obtained as such, or after concentration by evaporation of a medium with a lower concentration.

The propionate, succinate, and if present acetate, may be present in the acid form (counterion H), in the form of a salt, or as a mixture of acid and salt. Where the carboxylic acids is present in the form of a salt, it is preferred for the salt of sodium, potassium, magnesium or calcium. It is particularly preferred for the propionate, succinate, and if present acetate to be present in the form of a calcium salt. The ratio of acid to salt depends on the pH of the aqueous medium. The pH generally is in the range of 5 to 9, more specifically in the range of 6 to 8.

The aqueous medium can be obtained from various sources. In one embodiment it is obtained by subjecting a medium with a lower concentration to a concentration step, by evaporating water therefrom. The concentration step may, e.g., increase the propionate concentration with a factor 2 to 10, in particular with a factor 2 to 6. The concentration of the other compounds will of course increase proportionally. In one embodiment, the aqueous medium is obtained by concentrating a medium obtained from a fermentation process. This embodiment will be discussed in more detail below.

An inorganic soluble salt is provided in the aqueous medium in such an amount that the majority of succinate ions is converted to solid succinate salt while the majority of propionate ions remains in solution. In general, this means that a salt concentration of 5-15 wt.% is to be obtained. If the salt concentration is too low, the amount of succinate salt which precipitates is too low. On the other hand, if the salt concentration is too high, a propionate salt will also start to precipitate to an unacceptable amount.

There required salt concentration can be obtained in a number of manners.
In a first embodiment a salt or a salt solution is added to a solution comprising succinate and propionate until the required salt concentration is obtained. The salt may be added in the solid form. However, for ease of processing it may be preferred to add the salt in the form of an aqueous solution. The aqueous solution preferably has a relatively high concentration, e.g., of at least 10 wt.%, in particular at least 15 wt.%. The maximum is not critical and is determined by the solubility limit of the inorganic soluble salt.
In a second embodiment, a salt or a salt solution is added to a solution comprising succinate and propionate, and the resulting solution is concentrated by water removal until a solution is obtained wherein the salt concentration, succinate concentration, and propionate concentrations are sufficient to ensure that the majority of succinate ions is converted to solid succinate salt while the majority of propionate ions remains in solution. For more details on suitable salt concentration, succinate concentration, and propionate concentration, reference is made to what is stated above.

In one embodiment, the anion of the inorganic soluble salt is selected from chloride, nitrate, and hydroxide.
In one embodiment, the cation of the inorganic soluble salt is an alkaline earth metal cation, preferably Mg, or Ca, in particular Ca, or an alkaline metal cation, preferably K or Na. Of course, the nature of cation and anion should be matched so that a soluble salt is obtained. It is well within the scope of the skilled person to do this.
Suitable salts are calcium chloride and calcium nitrate, magnesium chloride, and magnesium nitrate, sodium chloride, sodium nitrate, and sodium hydroxide, and potassium chloride, potassium nitrate, and potassium hydroxide. The nitrate salts, in particular calcium nitrate and the chloride salts (calcium chloride, magnesium chloride, sodium chloride, and potassium chloride, in particular calcium chloride and magnesium chloride) may be particularly attractive from a performance point of view. The chloride salts may be attractive from a practical point of view.
In one embodiment, the cation of the inorganic soluble salt is the same as the cation of the propionate and succinate salt in the aqueous medium. The reason for this preference is to keep the number of different types of compounds as low as possible.

The majority of succinate ions is converted to solid succinate salt. Within the context of the present application, the wording "the majority" means at least 60%. It is preferred for at least 70% of the succinate ions to be converted to solid succinate salt, more preferably at least 85%, still more preferably at least 90%. It is particularly preferred for at least 95% of the succinate ions to be converted to solid succinate salt.

In this step, the majority, i.e. at least 60%, of propionate ions remains in solution. To have a high yield of solid propionate product, it is preferred for at least 70% of the propionate ions to remain in solution, more in particular at least 80%. On the other hand, as the aim of the process is to obtain a high-purity solid propionate salt, the focus in this step will generally be on the removal of succinate ions, and the process conditions adapted thereto. The propionate content of the aqueous medium will generally be in the range of present 8-30 wt.%, preferably in the range of 8-25 wt.%.

The aqueous medium comprising the majority of propionate ions is then separated from the solid succinate salt. This separation can be carried out through methods known in the art, including one or more of filtration, centrifugation, decantation, etc. It is within the scope of the skilled person to select a suitable manner.

The separation step yields a solid succinate salt fraction and an aqueous medium comprising propionate ions an inorganic salt. For the composition of the aqueous medium reference is made to what is stated above in the discussion of the aqueous phase before the removal of the succinate salt.

The solid succinate salt may be processed as desired. If so desired, it can be subjected to washing step, or a recrystallization step. Conventional purification methods like carbon treatment or ion exchange of a succinate salt solution may also be applied. The succinate salts can conveniently be converted to succinic acid by acidulation of a succinic acid salt solution, e.g., using HCl.

The aqueous medium comprising propionate ions an inorganic salt is further processed by increasing the concentration of salt in the aqueous medium to convert the majority of propionate ions to solid propionate salt.
The majority of propionate ions is converted to solid propionate salt. Within the context of the present application, the wording "the majority" means at least 60%. It is preferred for at least 70% of the propionate ions to be converted to solid propionate salt, more preferably at least 85%, still more preferably at least 90%. It is particularly preferred for at least 95% of the propionate ions to be converted to solid propionate salt.
The concentration of salt in the aqueous medium can be increased by adding an inorganic soluble salt to the aqueous medium, by subjecting the medium to an evaporation step to effect water removal, or by a combination of these two methods. It may be preferred for at least part of the concentration increase to be obtained by the evaporation of water.

Where an inorganic soluble salt is added to the aqueous medium, the considerations for the salt and its manner of addition are the same as given above for the addition of an inorganic soluble salt to the aqueous medium comprising succinate and propionate. The preferences expressed there also apply here.
It is preferred for the inorganic salt applied in this step to be the same as the inorganic salt applied in the step of adding an inorganic soluble salt to the aqueous medium comprising succinate and propionate.
The salt concentration is increased to a value where precipitation of the propionate salt will occur. When this will take place will depend on factors like the temperature and the propionate concentration. In one embodiment, the inorganic salt concentration is increased to a value of at least 15 wt.%, in particular at least 20 wt.%, more in particular at least 30 wt.%. As a maximum, a value of 40 wt.% may be mentioned. The maximum is defined by solubility considerations. The propionate concentration may be, e.g., at least 10 wt.%, in particular at least 15 wt.%, more in particular at least 20 wt.%. As a maximum, a value of 35 wt.% may be mentioned, depending on solubility considerations. It is within the scope of the skilled person to determine when the inorganic salt concentration has increased to such a value that precipitation of the propionate salt will take place (without substantial precipitation of the inorganic salt).

The solid propionate salt is separated from the aqueous medium. This can be done by methods known in the art, including one or more of filtration, centrifugation, decantation, etc. It is within the scope of the skilled person to select a suitable manner. If so desired, the propionate salt can be washed to increase product purity. The washing liquid, if used, can, e.g., be water or a propionate e salt solution. The washing liquid can be recycled to obtain a high yield.
The process according to the invention yields a solid propionate salt. The propionate salt preferably is magnesium propionate, calcium propionate, sodium propionate, or potassium propionate.
It is possible with the process according to the invention to prepare high-purity propionate salts. In one embodiment, the propionate salt has a succinate concentration which is below 8 wt.%, in particular below 5 wt.%, more in particular below 3 wt.%, or even below 1 wt.%.

One feature of the present invention is that organic contaminants present in the starting solution will not end up, or end up only to a limited extent, in the final propionate salt. There contaminants will remain in the aqueous medium. Examples of contaminants are of course succinate salts, which are precipitated, but also acetate salts, which remain in the aqueous medium upon precipitation of the succinate and the propionate. Further contaminants include remnants from a fermentation step.
The propionate salt, in particular calcium propionate, obtained by the process according to the invention preferably has a purity of at least 90 wt.%, in particular at least 95 wt.%, more in particular at least 97 wt.%.
The propionate salt can be used as desired, e.g., in the preservation of feed and food products. It can be converted into other products as desired.

In one embodiment, the propionate and succinate in the starting aqueous medium, the inorganic soluble salt added to that medium, and, if used, the inorganic salt added to the medium comprising propionate after the succinate salt has been separated therefrom all share the same cation, e.g., magnesium, calcium, potassium, or sodium, in particular calcium.
For example, in one embodiment, the process according to the invention encompasses the steps of
- providing an aqueous medium comprising dissolved calcium succinate and dissolved calcium propionate,
- providing an inorganic soluble calcium salt in the aqueous medium in such an amount that the majority of succinate ions is converted to solid calcium succinate while the majority of propionate ions remains in solution,
- separating the aqueous medium comprising the majority of propionate ions from the solid calcium succinate,
- increasing the concentration of calcium salt in the aqueous medium to convert the majority of propionate ions to solid calcium propionate,
- separating the solid calcium propionate from the aqueous medium.

As indicated above, the process of the present invention is particularly suitable for the treatment of aqueous media comprising propionate and succinate derived from a fermentation process. In one embodiment the process according to the invention therefore encompasses the steps of
- contacting a carbon source under fermentation conditions with a microorganism capable of generating propionic acid and succinic acid in an aqueous medium, wherein a base is added during the fermentation, to form a fermentation product comprising propionate and succinate, and
- subjecting the fermentation product to a biomass removal step.

In the first step a carbon source is subjected to a fermentation step to form propionate and succinate. Fermentation processes for the manufacture of propionate and succinate are known in the art and require no further elucidation here. It is within the scope of the skilled person to select, using his common general knowledge, a suitable fermentation process, including fermentation conditions, a suitable microorganism, and a suitable carbon source.
As is conventional in the art, a base is added during the fermentation step to compensate for the pH decrease which would occur due to the production of the carboxylic acids. Suitable bases include hydroxides, oxides, and carbonates of calcium, magnesium, sodium, and potassium, resulting in the formation of a fermentation broth containing respectively calcium succinate and propionate, magnesium succinate and propionate, sodium succinate and propionate, or potassium succinate and propionate.
In addition to the propionate and succinate, the fermentation product may optionally comprise further components such as impurities like are sugars, proteins, and salts.
The fermentation product is subjected to a biomass removal step. This may be carried out in manners known in the art, e.g., via filtration step. Efficient biomass removal will improve product quality, including product color.
In one embodiment, the process according to the invention encompasses the steps of preparing an aqueous medium comprising dissolved calcium succinate and dissolved calcium propionate by a process comprising the steps of
- contacting a carbon source under fermentation conditions with a microorganism capable of generating propionic acid and succinic acid in an aqueous medium, wherein a base is added during the fermentation, to form a fermentation product comprising propionate and succinate, the base being a calcium base,
- subjecting the fermentation product to a biomass removal step,
- subjecting the product of the biomass removal step to a concentration step.

Depending on the concentration of the propionate and succinate in the fermentation step, it may be preferred to subject the medium to a concentration step before it is treated in accordance with the process according to the invention. The concentration step can be carried out before, after, or simultaneous with biomass removal. Carrying out the concentration step after biomass removal is generally preferred. The concentration step can be carried out by methods known in the art, e.g., by evaporation of water. Other intermediate steps, e.g., purification steps such as with activated carbon, may be carried out as desired, as will be evident to the skilled person.

It will be clear to the skilled person that preferred embodiments of the various process steps can be combined.

The invention will be elucidated with reference to the following examples, without being limited thereto or thereby.

### Example 1

An aqueous medium comprising 5 wt.% of calcium propionate and 1 wt.% of calcium succinate was concentrated by evaporating water, to form an aqueous medium comprising 20 wt.% of calcium propionate and 4 wt.% calcium succinate. A 50 wt.% calcium chloride solution was added to the aqueous medium in such an amount that an aqueous medium was obtained comprising 8 wt.% of calcium chloride and 20 wt.% of calcium propionate. Calcium succinate had precipitated, and was removed by filtration. A minor amount (about 0.2 wt.%) of calcium succinate was still present in the solution.

To the aqueous medium comprising 8 wt.% of calcium chloride and 20 wt.% of calcium propionate, a 30 wt.% calcium chloride solution was added, until a 28 wt.% calcium chloride solution was obtained. At that point in time, calcium propionate had precipitated, and was removed from the reaction mixture by filtration.

### Example 2

2 wt.% of calcium chloride was added to an aqueous medium comprising 5 wt.% of calcium propionate and 1 wt.% calcium succinate. This medium was concentrated by evaporating water, to form an aqueous medium comprising 20 wt.% of calcium propionate, 4 wt.% calcium succinate and 8 wt.% of calcium chloride. Calcium succinate precipitated, and was removed by filtration. A minor amount (about 0.2 wt.%) of calcium succinate was still present in the solution.

The aqueous medium comprising 8 wt.% of calcium chloride and 20 wt.% of calcium propionate was further concentrated by evaporation to 30 wt.% calcium chloride. At that point in time, calcium propionate had precipitated, and was removed from the reaction mixture by filtration.

## Claims

1. Process for manufacturing a solid propionate salt from a mixture comprising propionate and succinate comprising the steps of
- providing an aqueous medium comprising succinate anions and propionate anions,
- providing an inorganic soluble salt in the aqueous medium in such an amount that the majority of succinate ions is converted to solid succinate salt while the majority of propionate ions remains in solution,
- separating the aqueous medium comprising the majority of propionate ions from the solid succinate salt,
- increasing the concentration of salt in the aqueous medium to convert the majority of propionate ions to solid propionate salt,
- separating the solid propionate salt from the aqueous medium.

2. Process according to claim 1, wherein the propionate concentration of the medium subjected to the succinate precipitation step is in the range of 15 to 25 wt.%, more preferably in the range of 18-23 wt.%.

3. Process according to claim 1 or 2, wherein the succinate concentration of the medium subjected to the succinate precipitation step is in the range of 0.5-10 wt.%, in particular 1-6 wt.%.

4. Process according to any one of the preceding claims wherein the step of providing an inorganic soluble salt in the aqueous medium in such an amount that the majority of succinate ions is converted to solid succinate salt is carried out by adding salt or a salt solution to a medium comprising succinate and propionate until the required salt concentration is obtained.

5. Process according to any one of claims 1-3, wherein the step of providing an inorganic soluble salt in the aqueous medium in such an amount that the majority of succinate ions is converted to solid succinate salt is carried out by adding a salt or a salt solution a solution comprising succinate and propionate, and concentrating the resulting solution by water removal until a solution is obtained wherein the salt concentration, succinate concentration, and propionate concentrations are sufficient to ensure that the majority of succinate ions is converted to solid succinate salt while the majority of propionate ions remains in solution.

6. Process according to any one of the preceding claims, wherein the concentration of salt in the aqueous medium to convert the majority of propionate ions to solid propionate salt is increased by adding an inorganic soluble salt to the aqueous medium.

7. Process according to any one of the preceding claims, wherein the concentration of salt in the aqueous medium to convert the majority of propionate ions to solid propionate salt is increased by subjecting the medium to an evaporation step to effect water removal.

8. Process according to any one of the preceding claims wherein the cation of the inorganic soluble salt is an alkaline earth metal cation, preferably Mg, or Ca, in particular Ca, or an alkaline metal cation, preferably K or Na.

9. Process according to any one of the preceding claims wherein the anion of the inorganic soluble salt is selected from chloride, nitrate, and hydroxide.

10. Process according to any one of the preceding claims wherein the propionate and succinate in the starting aqueous medium, the inorganic soluble salt added to that medium, and, if used, the inorganic salt added to the medium comprising propionate after the succinate salt has been separated therefrom all share the same cation, e.g., magnesium, calcium, potassium, or sodium, in particular calcium.

11. Process according to any one of the preceding claims which further comprises preparing an aqueous medium comprising succinate anions and propionate anions by a process comprising the steps of
- contacting a carbon source under fermentation conditions with a microorganism capable of generating propionic acid and succinic acid in an aqueous medium, wherein a base is added during the fermentation, to form a fermentation product comprising propionate and succinate,
- subjecting the fermentation product to a biomass removal step,
- subjecting the product of the biomass removal step to a concentration step.

12. Process according to claim 10, wherein the base added during fermentation has the same cation as the soluble inorganic salt.

13. Process according any one of the preceding claims, which comprises the steps of steps of
- providing an aqueous medium comprising dissolved calcium succinate and dissolved calcium propionate,
- providing an inorganic soluble calcium salt in the aqueous medium in such an amount that the majority of succinate ions is converted to solid calcium succinate while the majority of propionate ions remains in solution,
- separating the aqueous medium comprising the majority of propionate ions from the solid calcium succinate,
- increasing the concentration of calcium salt in the aqueous medium to convert the majority of propionate ions to solid calcium propionate,
- separating the solid calcium propionate from the aqueous medium.

14. Process according to claim 12, which further comprises preparing an aqueous medium comprising dissolved calcium succinate and dissolved calcium propionate by a process comprising the steps of
- contacting a carbon source under fermentation conditions with a microorganism capable of generating propionic acid and succinic acid in an aqueous medium, wherein a base is added during the fermentation, to form a fermentation product comprising propionate and succinate, the base being a calcium base,
- subjecting the fermentation product to a biomass removal step,
- subjecting the product of the biomass removal step to a concentration step.
